Europäisches Patentamt

European Patent Office

Office européen des brevets

(19)

(11) Veröffentlichungsnummer: **0 085 931**
**B1**

(12) **EUROPÄISCHE PATENTSCHRIFT**

(45) Veröffentlichungstag der Patentschrift:
30.04.86

(51) Int. Cl.⁴: **A 61 B 17/10**

(21) Anmeldenummer: **83100952.7**

(22) Anmeldetag: **02.02.83**

(54) **Chirurgisches Hautklammergerät.**

(30) Priorität: **10.02.82 DE 3204522**

(43) Veröffentlichungstag der Anmeldung:
**17.08.83 Patentblatt 83/33**

(45) Bekanntmachung des Hinweises auf die Patenterteilung:
**30.04.86 Patentblatt 86/18**

(84) Benannte Vertragsstaaten:
**AT BE CH DE FR GB IT LI LU NL SE**

(56) Entgegenhaltungen:
**EP - A - 0 040 157**
**EP - A - 0 069 557**
**WO - A - 81/02269**
**DE - A - 2 348 670**
**DE - A - 3 035 390**
**US - A - 4 364 507**

(73) Patentinhaber: **INTERMEDICAT GMBH,**
**Gerliswilstrasse 74, CH-6020 Emmenbrücke (CH)**

(72) Erfinder: **Braun, Karl, Oberer Brühl 5, D-7201 Talheim (DE)**
Erfinder: **Fetzer, Jürgen, Mühlstrasse 19, D-7921 Gerstetten-Dettingen (DE)**

(74) Vertreter: **von Kreisler, Alek, Dipl.-Chem. et al, Deichmannhaus am Hauptbahnhof, D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

## Beschreibung

Die Erfindung betrifft ein chirurgisches Hautklammergerät mit einem einen Klammervorrat enthaltenden Klammermagazin, einer Feder zum Nachschieben der Klammern in dem Klammermagazin, einer an der Gerätespitze vorgesehenen Amboßnase und mit einem relativ zu der Amboßnase in einem Kanal verschiebbaren Prägestößel zum Verformen einer an der Amboßnase abgestützten Klammer, bei welchem die Amboßnase quer zu dem Kanal zwischen einer Arbeitsstellung und einer Rückzugsstellung bewegbar ist und die Bewegung der Amboßnase in Abhängigkeit von der Stellung des Prägestößels in dem Kanal gesteuert ist.

Derartige Hautklammergeräte werden dazu benutzt, die adaptierten Wundränder von Hautteilen eines Patienten durch metallische Klammern zu verbinden. Durch Betätigen eines Prägehebels wird die Hautklammer von einem Prägestößel gegen eine an der Gerätespitze vorgesehene Amboßnase gedrückt und dabei verformt, so daß die aus der Gerätespitze herausragenden Teile der Klammer aufeinander zu bewegt werden und dabei in die Haut des Patienten eindringen.

Bei einem bekannten Hautklammergerät der eingangs genannten Art (WO-A-81/02269) werden die Klammern entlang eines geradlinigen Klammermagazins vorgeschoben. Die vorderste Klammer befindet sich in der Bewegungsbahn des Prägestößels. Diese Bewegungsbahn verläuft unter einem Winkel von 90° zur Richtung des Klammermagazins. Der Stößel ist hierbei am Ende eines langen Hebels angebracht, der sich quer zur Bewegungsrichtung des Stößels erstreckt und dessen anderes Ende mit einem Auslösehebel gekoppelt und in einer komplizierten Führungsbahn geführt ist. Der Auslösehebel steuert außerdem mehrere quer zu dem Kanal verlaufende Stangen, die während des Arbeitshubes des Stößels quer in den Kanal hineinragen und als Amboß für die Verformung der Klammer wirken. Nach Beendigung des Klammervorgangs werden die Stangen wieder zurückgezogen, sobald der unter der Wirkung einer Feder stehende Abzugshebel sich in seiner Ruhelage zurückbewegt. Dieses Hautklammergerät benötigt eine komplizierte Steuerung des Stößels über einen langen Hebel. Die Bewegungen von Stößel und Amboß sind nicht ohne weiteres zu koordinieren, da die beiden genannten Elemente über den Abzugshebel miteinander gekoppelt sind.

Bekannt ist ferner ein chirurgisches Hautklammergerät (EP-A-0 040 157), bei dem die Klammern gegen eine feste Amboßnase gedrückt werden, von der sie nach Beendigung der Verformung abgezogen werden. Das Klammermagazin erstreckt sich parallel zu dem Kanal, in dem der Stößel bewegt wird und mündet über eine Umbiegung rechtwinkig in diesen Kanal ein. Nach beendetem Klammervorgang muß die fest an der Gerätespitze angebracht Amboßnase aus der Klammer herausgezogen werden. Hierbei besteht die Gefahr, daß die geschlossene Klammer durch unachtsames Absetzen des Hautklammergerätes aus dem Gewebe herausgerissen wird.

Der Erfindung liegt die Aufgabe zugrunde, ein chirurgisches Hautklammergerät der eingangs genannten Art zu schaffen, bei dem die Steuerung der Amboßnase mit einfachen mechanischen Mitteln, die wenig Platz beanspruchen, erfolgt.

Zur Lösung dieser Aufgabe ist erfindungsgemäß vorgesehen, daß die Amboßnase an einer in dem Kanal verlaufenden Blattfeder befestigt ist, deren Spannung die Amboßnase in die Rückzugsstellung treibt und die eine mit einem Ansatz des Prägestößels zusammenwirkende Schrägfläche aufweist, und daß der Ansatz bei der Vorschubbewegung des Prägestößels auf die Schrägfläche aufläuft und dadurch die Blattfeder derart verformt, daß die Amboßnase in die Arbeitsstellung bewegt wird.

Hierbei ist die Bewegung der Amboßnasse mit derjenigen des Prägestößels gekoppelt. Wenn der Prägestößel sich in seine Arbeitsstellung bewegt, wird die Amboßnase selbsttätig ebenfalls in die Arbeitsstellung gebracht, in der sie in das Ende des Kanals, in dem sich der Prägestößel bewegt, hinein vorsteht. Die Bewegung der Klammer wird nun von der Amboßnase aufgehalten, wobei die Verformung zwischen Amboßnase und Prägestößel erfolgt. Wird der Prägestößel anschließend zurückgezogen, dann bewegt sich auch die Amboßnase selbsttätig in ihre Rückzugsstellung, so daß das Gerät ohne Behinderung von der geschlossenen Klammer abgenommen werden kann. Wichtig ist, daß die Amboßnase zusammen mit dem Präge-stößel in die Arbeitsstellung gebracht wird, wobei sich der Prägestößel in Längsrichtung des Kanales, die Amboßnase aber quer zum Kanal bewegt. Wenn der Ansatz des Prägestößels auf die Schrägfläche des Blattfeder aufläuft, wird die Blattfeder derart verformt, daß die Amboßnase in die Arbeitsstellung geschwenkt wird. Die Spannung der Blattfeder treibt die Amboßnase also in die Rückzugsstellung. Gemäß der Erfindung ist die Amboßnase an einer in dem Kanal verlaufenden Blattfeder befestigt, die eine mit einem Ansatz des Präge-stößels zusammenwirkende Schrägfläche aufweist. Wenn der Ansatz des Prägestößels auf die Schrägfläche der Blattfeder aufläuft, wird die Blattfeder derart verformt, daß die Amboßnase in die Arbeitsstellung geschwenkt wird. Die Spannung der Blattfeder treibt die Amboßnase also in die Rückzugsstellung. Bei Hautklammergeräten ist eine exakte Führung der Klammer während des Schließvorganges sehr wichtig, weil die Klammer in diesem Stadium an der Gerätespitze aus dem Kanal heraustritt. Gemäß einer bevorzugten Weiterbildung der Erfindung weist die Amboßnase in ihrer Mitte eine Kerbe oder einen Schlitz auf, um das Basisteil der Klammer während des Verformungsvorgangs festzuhalten. Eine symmetrische Ausbauchung der Klammer dringt in der Anfangsphase der Verformung in die Kerbe bzw. den Schlitz ein, so daß die Klammer sich anschließend nicht mehr drehen kann. Die Kerbränder bzw. Schlitzränder graben sich in das Material der Klammer ein und bewirken eine gewisse Verhakung im Mittelteil des Basisbereichs der Klammer. Gemäß einer bevorzugten Ausführungsform der Erfindung weist der Kanal seitliche sowie obere und untere Führungsflächen auf, die die Bewegungsbahn der in dem Kanal liegend vorgeschobenen vordersten Klammer begrenzen. Die Führungsflächen sind bis über die Amboßnase vorgezogen. Eine Formeinprägung fixiert die vorderste Klammer in dem Kanal. Beim Vorschieben des Prägestößels wird die Klammer zum Amboß transportiert, wobei die Zuführung weiterer Klammern aus dem Klammermagazin gesperrt wird. Durch die Führungsflächen erfolgt ein definierter Vorschub der Klammer in dem Kanal. Vorzugsweise sind die Führungsflächen in über die Amboßnase hinaus nach vorne vorstehenden Ansätzen fortgesetzt. Diese Ansätze haben relativ kleine Abmessungen. Die Handhabung des Klammergerätes wird erleichtert, wenn ein zweiteiliges Gehäuse vorgesehen ist, das einen einen Betätigungsmechanismus enthaltenden rückwärtigen Gehäuseteil und einen den Prägestößel und die Amboßnase enthaltenden, gegenüber dem rückwärtigen Gehäuseteil drehbaren vorderen Gehäuseteil aufweist. Durch die Drehbarkeit des vorderen Gehäuseteiles relativ zu dem rückwärtigen Gehäuseteil wird erreicht, daß die Ausrichtung der Klammer in Bezug auf den Betätigungsmechanismus frei gewählt werden kann. Der Arzt braucht also den Betätigungsmechanismus nicht quer zu der Hautnaht auszurichten, sondern er kann das Gerät in der jeweils für die Bedienung günstigsten Drehstellung halten. Von besonderer Wichtigkeit bei Hautklammern ist die Betätigung mit einem geringen Kraftaufwand. Nur wenn das Verformen und Setzen der Klammer mit geringer Körperkraft möglich ist, kann das Gerät mit der für ein präzises Setzen der Klammer erforderlichen Ruhe und Sicherheit gehalten werden. Um dies zu erreichen, ist der Betätigungshebel derart mit einem Griffteil gekoppelt, daß der wirksame Momentenarm des Betätigungshebels sich mit zunehmender Entfernung des Griffteiles aus seiner Ruhelage vergrößert, derart daß bei gleichbleibender Betätigungskraft die auf den Schieber einwirkende Vorschubkraft sich erhöht. In der ersten Phase der Betätigung des Griffteils wird die vorderste Klammer des Klammermagazins lediglich in dem Kanal vorgeschoben und gegen die Amboßnase gesetzt. In dieser Phase ist der erforderliche Kraftaufwand relativ gering, so daß die Hebelübersetzung nicht notwendigerweise optimiert werden muß. Der Momentenarm der Hebelübersetzung erreicht jedoch sein Maximum in der Endphase der Verschiebungsbewegung, nämlich dann,wenn die Verformung der Klammer erfolgt. Der für die Verformung benötigte Kraftaufwand wird durch die Hebelübertragung derart herabgesetzt, daß es möglich ist, die Verformung der Klammer herbeizuführen, indem das Griffteil lediglich mit dem Zeigefinger bewegt wird. Die Betätigungskraft ist gegenüber den bekannten Klammergeräten auf etwa die Hälfte herabgesetzt. Prinzipiell ist es möglich, die Bewegung des Präge-stößels mit der Längsbewegung des Schiebers zwangsweise zu koppeln. Dies hätte jedoch den Nachteil, daß der Präge-stößel jede Bewegung des Schiebers mitmachen würde. Wenn der Schieber nicht bis zu seinem vorderen Ende vorgeschoben wird, würde die Gefahr bestehen, daß eine zweite Klammer in den Kanal des Prägestößels gelangt, ohne daß die erste Klammer verformt und freigegeben wurde. Um dies zu vermeiden, ist gemäß einer bevorzugten Weiterbildung der Erfindung vorgesehen, daß der Schieber eine in Querrichtung federnde Zunge aufweist, die mit einem Steuernocken des Gehäuses zusammenwirkt und eine gegen eine Querkante des Prägestößels ansetzbare Nase aufweist. Die Nase des Schiebers zieht den Prägestößel nur dann zurück, wenn dieser seine vordere Endstellung erreicht hat. Nur in diesem Fall wird die Einmündung des Klammermagazins in den Kanal freigegeben, so daß die nächstfolgende Klammer nachgeschoben werden kann. Gemäß einer bevorzugten Ausführungsform der Erfindung weist die Zunge einen ein am Gehäuse angebrachtes Zählwerk fortschaltenden Ansatz auf. An dem Zählwerk kann die Anzahl der verbrauchten oder die Anzahl der noch im Klammermagazin vorhandenen Klammern abgelesen werden. Im Folgenden wird unter Bezugnahme auf die Zeichnungen ein Ausführungsbeispiel der Erfindung näher erläutert. Es zeigen:

Fig. 1 einen schematischen Längsschnitt durch das Hautklammergerät,

Fig. 2 einen Längsschnitt durch die Gerätespitze bei zurückgezogener Amboßnase,

Fig. 3 eine ähnliche Darstellung wie Fig. 2, wobei sich jedoch die Amboßnase in der Arbeitsstellung befindet,

Fig. 4 einen Schnitt entlang der Linie IV-IV von Fig. 2,

Fig. 5 einen Schnitt entlang der Linie V-V von Fig. 3,

Fig. 6 einen Vertikalschnitt durch das Klammermagazin,

Fig. 7 das Zusammenwirken des Schiebers und des Prägestößels während der Vorschubbewegung,

Fig. 8 das Zusammenwirken des Schiebers und des Präge-stößels kurz vor der Rückzugsbewegung,

Fig. 9 eine Draufsicht von Fig. 8,

Fig. 10 einen Vertikalschnitt durch eine modifizierte Ausführungsform der Gerätespitze mit Zählwerk,

Fig. 11 eine modifizierte Ausführungsform des Betätigungsmechanismus in der Rückzugsstellung des Schiebers, und

Fig. 12 den Betätigungsmechanismus nach Fig. 11 in der Vorschubstellung des Schiebers.

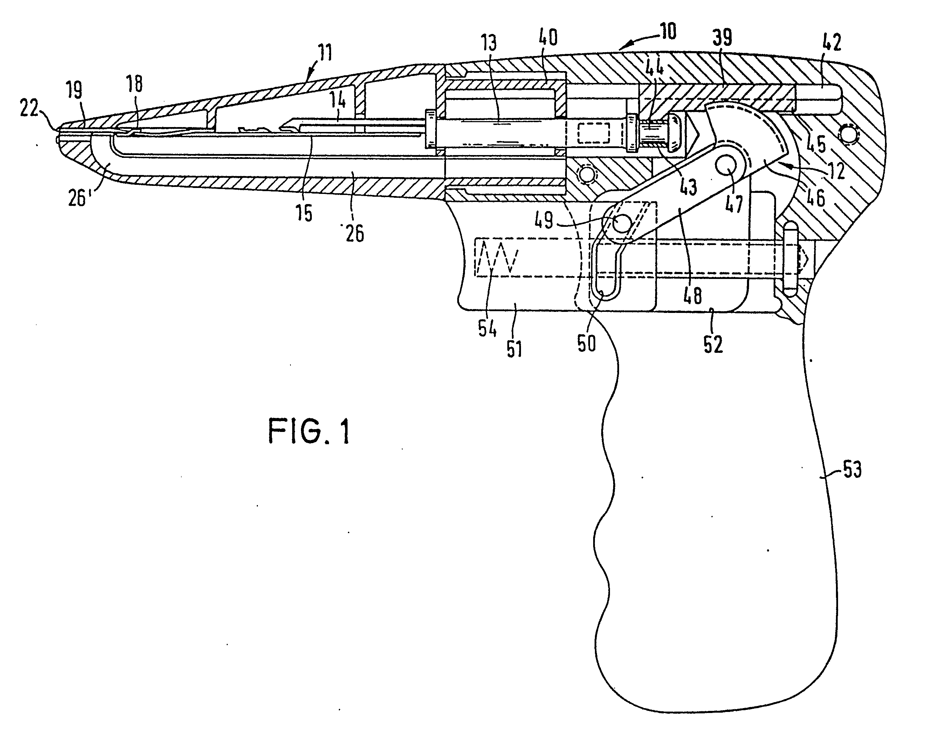

Das in den Fig. 1 bis 9 dargestellte Hautklammergerät weist einen rückwärtigen Gehäuseteil 10 und einen vorderen Gehäuseteil 11 auf. Der vordere Gehäuseteil 11 ist in dem den Betätigungsmechanismus 12 enthaltenden rückwärtigen Gehäuseteil 10 um seine Längsachse drehbar gelagert. In dem vorderen Gehäuseteil 11 ist ein Schieber 13 längsverschiebbar, jedoch unverdrehbar geführt. Der Schieber 13 weist eine nach vorne abstehende elastische Zunge 14 auf, die mit dem Prägestößel 15 zusammenwirkt. Der Prägestößel 15 besteht aus einem langgestreckten starren Materialstreifen, der in dem Kanal 16 in Längsrichtung verschiebbar angeordnet ist. Der Materialstreifen weist eine mittlere Ausnehmung 17 auf. Am vorderen Ende dieser Ausnehmung 17 befindet sich eine hochgebogene Nase 18 mit einem verbreiterten Kopf.

In dem Kanal 16 verläuft ferner eine Blattfeder 19 im wesentlichen parallel zu dem Prägestößel 15. Die Blattfeder 19 weist eine Schrägfläche 20 auf und ist mit einem allseitig begrenzten mittleren Schlitz 21 versehen, der sich über den Bereich der Schrägfläche 20 nach vorne und hinten erstreckt. Durch den Schlitz 21 ragt die Nase 18 des Prägestößels 15 hindurch. Der verbreiterte Kopf dieser Nase 18 drückt gegen die Oberseite der Blattfeder 19. Das vordere Ende der Blattfeder 19 ist nach unten umgebogen und bildet die Amboßnase 22. Das rückwärtige Ende der Blattfeder 19 ist an dem vorderen Gehäuseteil 11 fixiert.

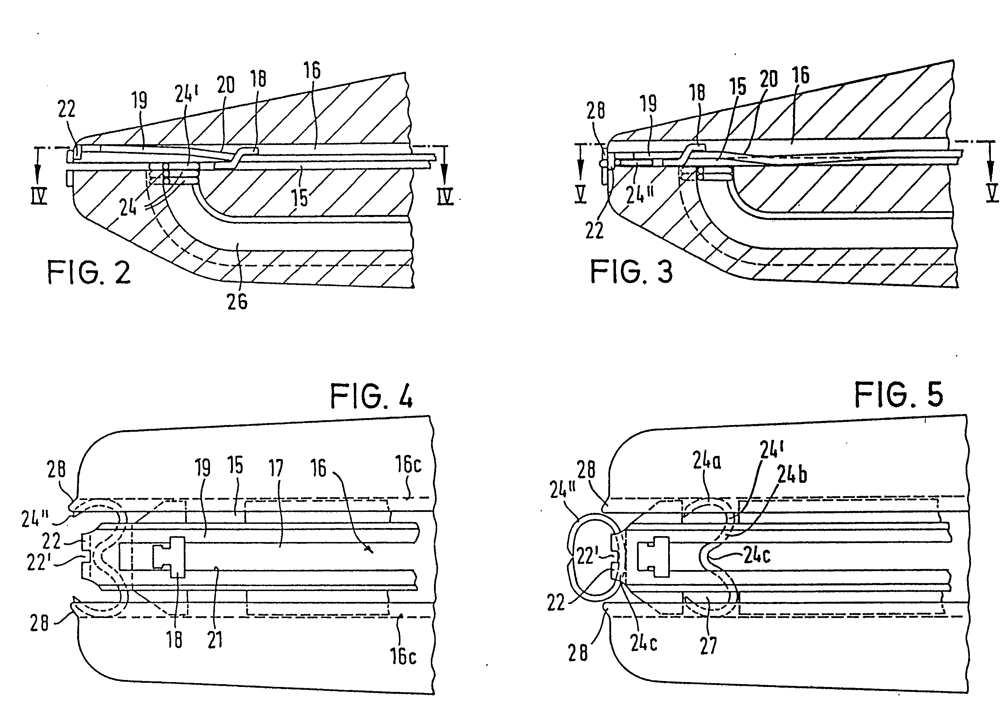

Wenn der Prägestößel 15 sich in der in Fig. 2 dargestellten Rückzugsstellung befindet, liegt die Nase 18 am Fuße der Schrägfläche 20. Dies bedeutet, daß der vordere Bereich der Blattfeder 19 sich infolge seiner Eigenspannung in dem Kanal 16 gemäß Fig. 2 hochstellt. Da die Höhe des Kanals 16 größer ist als die Höhe der Amboßnase 22, gibt die zurückgezogene Amboßnase 22 den unteren Bereich 16' des Kanals 16 nach vorne hin frei.

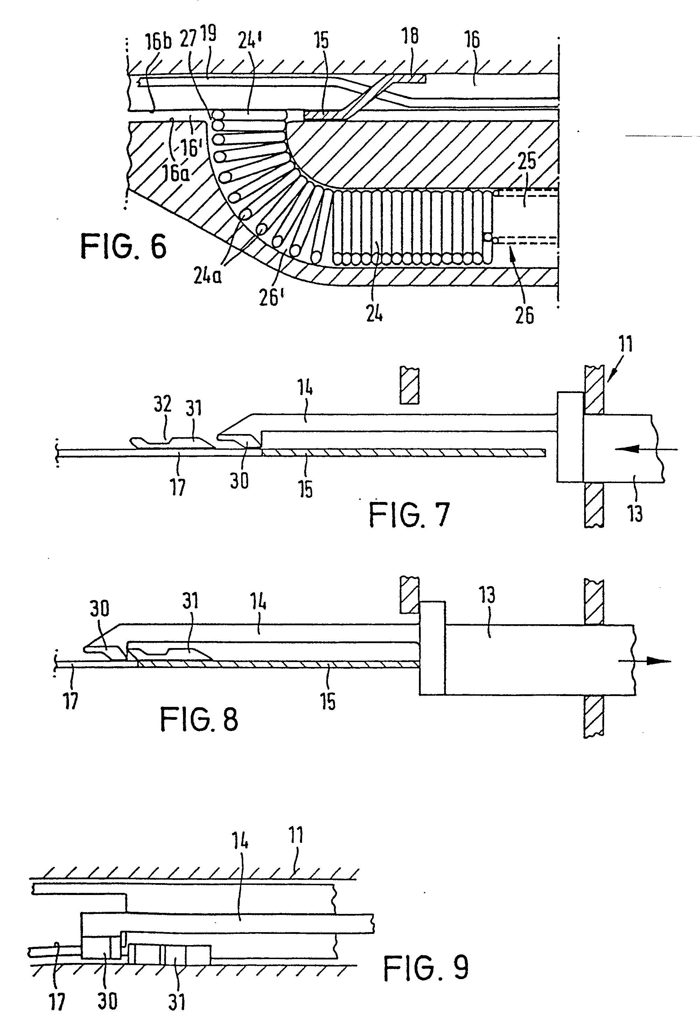

Parallel zu dem Kanal 16 verläuft das Klammermagazin 26 Das Klammermagazin ist ein in dem vorderen Gehäuseteil 11 vorgesehener Kanal, in welchem die Klammern 24 Seite an Seite stehend angeordnet sind. Eine Schraubenfeder 25 (Fig. 6), die sich an dem Gehäuse abstützt, drückt gegen die rückwärtige Klammer 24 und schiebt den gesamten Klammerstapel unter ständiger Vorspannung vor. Das vordere Ende des Klammermagazins 26 ist in einem Bogen von 90° ausgeführt. Dieses Ende des Klammermagazins mündet in den Kanal 16, so daß die vorderste Klammer 24' von der Feder 25 in den Kanal 16 hineingedrückt wird. Der untere Bereich 16' des Kanals 16 hat eine größere Breite als der darüberliegende Bereich, in dem sich die Blattfeder 19 vertikal bewegen kann. Die Höhe des unteren Führungskanals 16' ist nur geringfügig größer als die Stärke der Klammern 24'. Der Bereich 16' bildet einen Führungskanal zum Vorschieben der vordersten Klammer 24' und zur Führung des Prägestößels 15. Dieser Führungskanal ist durch die untere Führungsfläche 16a, die beiden oberen Führungsflächen 16b (Fig. 6) und durch die seitlichen Führungsflächen 16c (Fig. 4) begrenzt.

Die Klammern 24, deren Form im Ausgangszustand aus Fig. 5 ersichtlich ist, haben bogenförmige Beine 24a, die jeweils über einen schräg nach innen und vorne gerichteten geradlinigen Bereich 24b in das Basisteil 24c übergehen. Das Basisteil 24c besteht aus einem nach vorne gebogenen Halbkreis, dessen Scheitel sich beim Vorschieben gegen die Amboßnase 22 legt. Zur besseren Zentrierung der Klammer 24 ist die Amboßnase 22 mit einem vertikalen Schlitz 22' versehen.

In dem bogenförmigen Bereich 26' des Klammermagazin 26 entfernen sich die Beine 24a benachbarter Klammern 24 voneinander, da die Beine 24a nach außen zeigen. Die Feder 25 drückt dagegen gegen denjenigen Bereich der Klammern 24', der in dem bogenförmigen Abschnitt 26' innen liegt.

An der Mündungsstelle 27, an der das Klammermagazin 26 in den Kanal 16 mündet, befinden sich an der Unterseite der oberen Führungsflächen 16b (nicht dargestellte) Formeinprägungen, um die vorderste Klammer 24' in einer definierten Position zu halten. Wird anschließend der Prägestößel 15 aus der in Fig. 2 gezeigten Rückzugsstellung vorgeschoben, dann stößt er mit seinem vorderen Ende gegen die vorderste Klammer 24' und schiebt diese in dem Kanalabschnitt 16' vor. Gleichzeitig schiebt sich die Nase 18 an der Schrägfläche 20 der Blattfeder 19 entlang, so daß die am vorderen Ende der Blattfeder 19 befindliche Amboßnase 22 von der Rückzugsstellung in die in Fig. 3 dargestellte Arteitsstellung gebracht wird. Die in den Fig. 3 und 4 mit 24'' bezeichnete Klammer befindet sich nunmehr zwischen dem vorderen Ende des Präge- stößels 15 und der Amboßnase 22 in einer Position, in der die Spitzen der Klammerbeine geringfügig nach vorne aus dem Gerät hervorstehen. Beim nachfolgenden Weiterschieben des Prägestößels 15 treten die Klammerbeine aus dem vorderen Ende des Gerätes heraus, wobei sich die Klammer 24'' in der in Fig. 5 gezeigten Weise schließt, während ihre Basis 24c an der Innenseite der Amboßnase 22 geradegebogen wird.

Um einen möglichst langen Führungsweg teim Verformen der Klammer 24'' zu erhalten, sind die Führungsflächen 16a, 16b und 16c bis in Ansätze 28 hinein fortgesetzt, die den Austrittsspalt des Kanals 16 aus dem Gehäuse begrenzen und geringfügig üter die Amtoßnase 22 hinaus vorstehen.

Sobald der Prägestößel 15 die vorderste Klammer 24' von der Mündungsstelle 27 fortgeschoben hat, wird die Mündungsstelle 27 von dem Prägestößel verschlossen, so daß die nächstfolgende Klammer noch nicht in den Kanal 16 vorgeschoben werden kann. Dies ist erst möglich, nachdem der Prägestößel 15 wieder in seine rückwärtige Endlage gebracht worden ist, in der er die Mündungsstelle 27 freigibt.

In den Fig. 7 und 8 ist die Steuerung des Prägestößels 15 durch den Schieter 13 dargestellt. Der Schieber 13, der in dem vorderen Gehäuseteil 11 längsverschiebbar, jedoch unverdrehtar gelagert ist, weist an seinem vorderen Ende die nach vorne abstehende und vertikal federnde Zunge 14 auf, an deren Ende sich ein seitlich abstehender Führungsflügel 30 befindet. Der Führunggflügel 30 arbeitet mit einem Steuernocken 31 des Gehäuseteils 11 zusammen.

Wird der Schieber 13 durch den Betätigungsmechanismus 12 vorgeschoben, dann stößt er mit seiner Stirnseite den Prägestößel 15 in Richtung auf die Gerätespitze. Durch die dem Flügel 30 angeformte vordere Schräge läuft der Flügel 31 auf eine rückwärtige Schräge des Steuernockens 31 auf. Die Zunge 14 weicht hierbei nach oben aus. Der Flügel 30 gleitet auf der oberen Nockenfläche 32. Wird der Schieber 13 zurückgezogen, bevor die vordere Endstellung erreicht ist, dann gleitet der Flügel 30 auf der oberen Nockenfläche 32 zurück, während der Prägestößel 15 die eingenommene Vorschutposition beibehält.

Erst wenn der Schieber 13 die in Fig. 8 dargestellte Position erreicht und der Flügel 30 über das vordere Ende der Steuernocken 31 hinweggegangen ist, ist der Prägevorgang beendet und die Klammer 24'' geschlossen. Bei der Zurückbewegung des Schiebers 13 gelangt der Flügel 30 mit seiner rückwärtigen Schrägfläche unter eine vordere Schrägfläche des Steuernockens 31. Daher wird die Zunge 14 nach unten gedrückt, wobei ein Vorsprung der Zunge 14 in den Längsschlitz 17 des Präge-stößels 15 gelangt und bei der weiteren Rückwärtsbewegung des Schieters 13 den Prägestößel 15 in die Ausgangsstellung zurückzieht. Solange der Prägestößel 15 nicht in die Ausgangsstellung zurückgezogen ist, gibt er die Mündungsstelle 27 des Klammermagazins 26 in den Kanal 16 nicht frei.

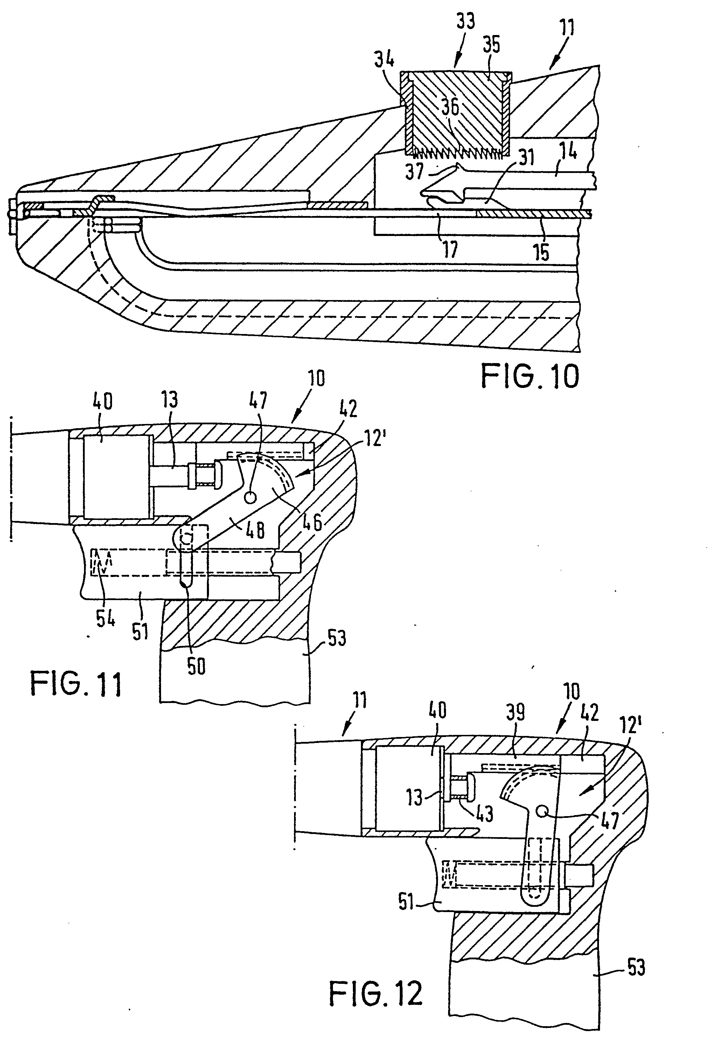

Fig. 10 zeigt ein zusätzlich an dem vorderen Gehäuseteil 11 angebrachtes Zählwerk 33, das von der Zunge 14 des Schiebers 13 fortgeschaltet wird. Das Zählwerk 33 besteht aus einem an dem Gehäuseteil 11 fest angebrachten Skalenring 34,in welchem eine Zahnscheibe 35 drehbar gelagert ist. An der Unterseite der Zahnscheibe 35 befindet sich ein Zahnkranz 36 aus Rastzähnen, in die ein Ansatz 37,der am vorderen Ende der Zunge 14 angebracht ist, eingreift, wenn der Flügel 30 bei der Vorschubbewegung von dem Führungsnocken 31 angehoben wird. Auf diese Weise wird die Zahnscheite 35 bei jeder Vorschubbewegung des Prägestößels 15 um einen bestimmten Winkel weitergedreht. An der oberen Stirnseite der Zahnscheibe 35 befindet sich eine Markierung, die an einer an dem Skalenring 34 vorgesehenen Skala die Anzahl der noch in dem Klammermagazin 26 enthaltenen Klammern 24 anzeigt.

Der vordere Gehäuseteil 11 weist an seinem rückwärtigen Ende eine zylindrische Buchse 40 auf, in der der Schieter 13 koaxial gelagert ist. Zum Auffüllen des Klammermagazins 26 kann die zylindrische Buchse 40 aus dem rückwärtigen Gehäuseteil 10 herausgezogen werden.

Das rückwärtige Ende des Schiebers 13 ist mit einem Schieteteil 39 gekoppelt, das längs einer Schienenführung 42 im Innern des rückwärtigen Gehäuseteils 10 bewegbar ist. Das Schiebeteil 39 weist eine Hülse 43 auf, durch die ein beidseitig durch Flansche begrenzter Schaft 44 des Schiebers 13 hindurchgeht. Das Schiebeteil 39 ist mit einer Zahnstange 45 versehen, in die die Verzahnung eines Zahnscheibensegmentes 46 eingreift. Das Zahnscheibensegment 46 bildet den einen Hebelarm eines zweiarmigen Hebels, der um eine Gelenkachse 47 des Gehäuseteils 10 schwenkbar ist. Der andere Hebelarm 48 greift mit einem Querstift 49 in einen Querschlitz 50 des Drückers 51 ein. Der Drücker 51 ist in einer etwa parallel zu dem Kanal 16 verlaufenden Ausnehmung 52 des Handgriffs 53 geführt und er wird von einer Feder 54 aus dem Handgriff 53 herausgedrückt. Der Drücker 51 ist so bemessen, daß er beim Ergreifen des Handgriffs 53 mit dem Zeigefinger betätigt werden kann. Wird er in den Handgriff 53 hineingedrückt, dann wird der Hebel 46, 48 um die Achse 47 herum verschwenkt, so daß das Schiebeteil 39 vorgeschoben wird und dabei den Schieber 13 ebenfalls nach vorne drückt. In der Nähe der Endstellung verläuft der Hebel 46,48 nahezu rechtwinklig zu dem Schieber 13, so daß der Momentenarm dann am größten ist.

Ein ähnlicher Betätigungsmechanismus 12' wie derjenige nach Fig. 1 ist in den Fig. 11 und 12 dargestellt, wobei in Fig. 11 die Rückzugsstellung und in Fig. 12 die Vorschubstellung des Schiebers 13 abgebildet ist. Die Feder 54 dient dazu, den Drücker 51 aus dem Handgriff 53 herauszudrücken und gleichzeitig das Schiebeteil 39 und damit auch den Schieber 13 in die Rückzugsstellung zu bringen. Während bei dem Ausführungsbeispiel der Fig. 1 der Querschlitz 50 des Drückers 51 einen abgewinkelten Verlauf hat, ist bei dem Ausführungsbeispiel der Fig. 11 und 12 der Querschlitz 50 geradlinig.

**Patentansprüche**

1. Chirurgisches Hautklammergerät mit einem einen Klammervorrat enthaltenden Klammermagazin (26), einer Feder (25) zum Nachschieben der Klammer in dem Klammermagazin, einer an der Gerätespitze vorgesehenen Amboßnase (22) und mit einem relativ zu der Amboßnase in einem Kanal verschiebbaren Präge-stößel (15) zum Verformen einer an der Amboßnase abgestützten Klammer,bei welchem die Amboßnase (22) quer zu dem Kanal (16) zwischen einer Arbeitsstellung und einer Rückzugsstellung bewegbar ist und die Bewegung der Amboßnase (22) in Abhängigkeit von der Stellung des Prägestößels (15) in dem Kanal (16) gesteuert ist,
dadurch gekennzeichnet,
daß die Amboßnase (22) an einer in dem Kanal (16) verlaufenden Blattfeder (19) befestigt ist, deren Spannung die Amboßnase (22) in die Rückzugsstellung treibt und die eine mit einem Ansatz (18) des Prägestößels (15) zusammenwirkende Schrägfläche (20) aufweist, und daß der Ansatz (18) bei der Vorschubbewegung des Prägestößels (15) auf die Schrägfläche (20) aufläuft und dadurch die Blattfeder (19) derart verformt, daß die Amboßnase (22) in die Arbeitsstellung bewegt wird.

2. Hautklammergerät nach Anspruch 1, dadurch gekennzeichnet, daß das Klammermagazin (26) im wesentlichen parallel zu dem Kanal (16) verläuft und an seinem vorderen Ende eine in den Kanal mündende Umbiegung (26') aufweist.

3. Hautklammergerät nach einem der Ansprüche 1 oder 2 dadurch gekennzeichnet, daß die Amboßnase (22) in ihrer Mitte eine Kerbe oder einen Schlitz (22') aufweist.

4. Chirurgisches Hautklammergerät nach einem der Ansprüche 1 bis 3, dadurch gekennzeichnet, daß der Kanal (16) seitliche sowie obere und untere Führungsflächen (16a, 16b) aufweist, die die Bewegungsbahn der in dem Kanal liegend vorgeschobenen vordersten Klammer (24') begrenzen und daß die Führungsflächen bis über die Amboßnase (22) vorgezogen sind.

5. Hautklammergerät nach Anspruch 4, dadurch gekennzeichnet, daß die Führungsflächen (16 a,b) in über die Amboßnase (22) hinaus nach vorne vorstehenden Ansätzen (28) fortgesetzt sind.

6. Hautklammergerät nach einem der Ansprüche 1 bis 5, dadurch gekennzeichnet, daß ein zweiteiliges Gehäuse (10,11) vorgesehen ist, das einen einen Betätigungsmechanismus (12) enthaltenden rückwärtigen Gehäuseteil (10) und einen Prägestößel (15) und die Amboßnase (22) enthaltenden, gegenüber dem rückwärtigen Gehäuseteil (10) drehbaren vorderen Gehäuseteil (11) aufweist.

7. Hautklammergerät nach Anspruch 6, dadurch gekennzeichnet, daß in dem vorderen Gehäuseteil (11) ein in Längsrichtung verschiebbarer Schieber (13) gelagert ist, dessen rückwärtiges Ende drehbar mit einem Schiebeteil (39) gekoppelt ist.

8. Hautklammergerät nach Anspruch 7, dadurch gekennzeichnet, daß das Schiebeteil (39) ein in einer Schienenführung (42) geführter Schlitten ist, der durch einen Betätigunashebel (46, 48) bewegbar ist.

9. Hautklammergerät nach Anspruch 8, dadurch gekennzeichnet, daß der Betätigungshebel (46,48) über ein Verzahnungssegment (46) mit einer Zahnstange (45) des Schlittens in Eingriff steht.

10. Hautklammergerät nach einem der vorhergehenden Ansprüche, dadurch gekennzeichnet, daß der Betätigungshebel (46, 48) derart mit einem Griffteil (51) gekoppelt ist, daß der wirksame Momentenarm des Betätigungshebels sich mit zunehmender Entfernung des Griffteiles aus seiner Ruhelage vergrößert, derart, daß bei gleichbleibender Betätigungskraft die auf den Schieber (13) einwirkende Vorschubkraft sich erhöht.

11. Hautklammergerät nach einem der vorhergehenden Ansprüche, dadurch gekennzeichnet, daß der Schieber (13) eine in Querrichtung federnde Zunge (14) aufweist, die mit einem Steuernocken (31) des Gehäuses (10,11) zusammenwirkt und eine gegen eine Querkante des prägestößels (15) ansetzbare Nase aufweist.

12. Hautklammergerät nach einem der Ansprüche 1 bis 11, dadurch gekennzeichnet, daß der Prägestößel (15) die Einmündung (27) des Klammermagazins (26) in den Kanal (16) nur in voll zurückgezogenem Zustand freigibt.

13. Hautklammergerät nach den Ansprüchen 11 und 12, dadurch gekennzeichnet, daß die Nase des Schiebers (13) den Prägestößel (15) nur dann zurückzieht, wenn dieser zuvor seine vordere Endstellung erreicht hat.

14. Hautklammergerät nach Anspruch 11, dadurch gekennzeichnet, daß die Zunge (14) einen ein am Gehäuse (10, 11) angebrachtes Zählwerk (33) fortschaltenden Ansatz (37) aufweist.

**Claims**

1. Surgical stapler comprising a staple supply magazine (26), a spring (25) for feeding the staples (24) in the magazine, an anvil nose (22) provided at the pointed end of the device and a deforming punch (15) displaceable in a channel (16) relative to the anvilnose(22)and adapted to deform a staple (24") backed by the anvil nose (22),
said anvil nose (22) being movable transversely to the channel (16) between a working position and a return position and the movement of the anvil nose (22) being controlled responsive to the position of the forming punch (15) in the channel (16),
characterized in that
the anvil nose (22) is fixed to a leaf spring (19) extending in the channel (16) and driving by its tension the anvil nose (22) into the return position, said spring including an inclined surface (20) cooperating with an attachment (18) of the forming punch (15), and that, in case of an advance movement of the forming punch (15), the attachment (18) moves up the inclined surface (20) to thus deforming the leaf spring (l9)so that the anvil nose (22) is urged into the working position.

2. Surgical stapler according to claim 1, characterized in that the staple magazine (26) extends substantia ly in parallel to the channel (16) and that its front end includes a deflection (26') ending in the channel.

3. Surgical stapler according to one of claims 1 or 2, characterized in the center of the anvil nose (22) contains a notch or slot (22').

4. Surgical stapler according to one of claims 1 to 3, characterized in that the channel (16) contains lateral upper and lower guide faces (l6a,l6b) limiting the travel path of the frontmost staple (24') horizontally fed in the channel and that the guide faces extend beyond the anvil nose (22)

5. Surgical stapler according to claim 4, characterized in that the guide faces (l6a,b) are continued in attachments (28) projecting forwardly beyond the anvil nose (22).

6. Surgical stapler according to one of claims 1 to 5, characterized by the provision of a bipartite housing (10, 11) which comprises a rearward housing portion (10) including an operating system (12) and a front housing portion (11) including the forming punch (15) and the anvil nose (10) and being rotatable relative to the rearward housing portion (10).

7. Surgical stapler according to claim 6, characterized in that in the fronthousing portion (11), a pusher (13) is mounted to be displaceable longitudinally and the rearward end of which is coupled rotatably to a pushing element (39).

8. Surgical stapler according to claim 7, characterized in that the pushing element (39) is a slide conducted in a rail guide (42)and movable by an actuating lever (46, 48).

9. Surgical stapler according to claim 8, characterized in that the actuating lever (46,48) engages via a toothed segment (14) a rack (45) of the slide.

10. Surgical stapler according to one of the preceding claims, characterized in that the actuating lever (46,48) is so coupled to a handle portion (51) that the effective arm of the actuating lever is increased as the handle portion is displaced from its rest position so that with a constant operating force, the advance force acting on the pusher (13) is intensified.

11. Surgical stapler according to one of the preceding claims characterized in that the pusher (13) comprises a transversely flexible tongue (14) coacting with the control cam (31) of the housing (10,11) and having a nose capable to engage a transverse edge of the forming punch (15).

12. Surgical stapler according to one of claims 1 to 11, characterized in that only in its fully withdrawn condition, the forming punch (15) allows access to the channel (16) for the mouth (27) of the staple magazine (26).

13. Surgical stapler according to one of claims 11 and 12, characterized in that the nose of the pusher (13) does not withdraw the forming punch (15) unless the latter previously reaches its front end position.

14. Surgical stapler according to claim 11, characterized in that the tongue (14) has an attachment (37) indexing a counter (33) mounted at the housing (10, 11).

**Revendications**

1. Agrafeuse chirurgicale comportant un magasin à agrafes (26) contenant une provision d'agrafes, un ressort (25) destiné à faire avancer les agrafes (24) dans ce magasin, un nez d'enclume (22) prévu à l'extrémité de l'agrafeuse, et un poussoir estampeur (15) mobile dans un canal (16) par rapport au nez d'enclume (22) et destiné à mettre en forme une agrafe (24'') appuyée sur le nez d'enclume (22), le nez d'enclume (22) étant mobile perpendiculairement au canal (16) entre une position de travail et une position reculée et son mouvement étant commandé en fonction de la position du poussoir estampeur (15) dans le canal (16), caractérisée en ce que le nez d'enclume (22) est fixé à un ressort lame (19) s'étendant dans le canal (16) dont la tension pousse le nez d'enclume (22) en position reculée et qui présente une surface inclinée (20) coopérant avec un appendice (18) du poussoir estampeur (15), et en ce que l'appendice (18), lors du mouvement d'avance du poussoir estampeur (15) monte sur la surface inclinée (20) et déforme par là le ressort lame (19) de façon telle que le nez d'enclume (22) soit amené en position de travail.

2. Agrafeuse selon la revendication 1, caractérisée en ce que le magasin à agrafes (26) s'étend de manière générale parallèlement au canal (16) et présente à son extrémité avant une partie courbe (26') qui débouche dans le canal.

3. Agrafeuse selon l'une des revendications 1 ou 2, caractérisée en ce que le nez d'enclume (22) présente au milieu une encoche ou une fente (22').

4. Agrafeuse chirurgicale selon l'une quelconque des revendications 1 à 3, caractérisée en ce que le canal (16) présente des surfaces de guidage latérales ainsi que supérieures et inférieures (16a, 16b) qui limitent la trajectoire de l'agrafe la plus en avant (24') avancée couchée dans le canal, et en ce que ces surfaces de guidage s'avancent au-delà du nez d'enclume (22).

5. Agrafeuse selon la revendication 4, caractérisée en ce que les surfaces de guidage (16a, 16b) se continuent sur des appendices (28) s'avançant au-delà du nez d'enclume (22).

6. Agrafeuse selon l'une quelconque des revendications 1 à 5, caractérisée en ce qu'il est prévu un corps (10, 11) en deux parties, une partie arrière (10) contenant un mécanisme de commande (12) et une partie avant (11) contenant un poussoir estampeur (15) et le nez d'enclume (22) et pouvant tourner par rapport à la partie arrière (10).

7. Agrafeuse selon la revendication 6, caractérisée en ce que dans la partie de corps avant (11) est monté un coulisseau (13) mobile dans la direction longitudinale dont l'extrémité arrière est accouplée de manière tournante à un élément coulissant (39).

8. Agrafeuse selon la revendication 7, caractérisée en ce que l'élément coulissant (39) est un chariot qui est guidé dans un guidage à rails (42) et peut être mû par un levier de commande (46, 48).

9. Agrafeuse selon la revendication 8, caractérisée en ce que le levier de commande (46, 48) est en prise par un secteur denté (46) avec une crémaillère (45) du chariot.

10. Agrafeuse selon l'une quelconque des revendications 1 à 9, caractérisée en ce que le levier de commande (46, 48) est accouplé à un organe de manoeuvre (51) de façon telle que le bras de moment effectif du levier de commande augmente quand l'organe de manoeuvre s'éloigne de sa position de repos, de façon telle qu'avec une force de commande constante la poussée sur le coulisseau (13) augmente.

11. Agrafeuse selon l'une quelconque des revendications 1 à 10, caractérisée en ce que le coulisseau (13) présente une languette (14) élastique dans la direction transversale qui coopère avec une came de commande (31) du corps (10, 11) et présente une saillie applicable contre un bord transversal du poussoir estampeur (15).

12. Agrafeuse selon l'une quelconque des revendications 1 à 11, caractérisée en ce que le poussoir estampeur (15) libère le débouché (27) du magasin à agrafes (26) dans le canal (16) seulement à l'état complètement reculé.

13. Agrafeuse selon l'une des revendications 11 ou 12, caractérisée en ce que la saillie du coulisseau (13) ne fait reculer le poussoir estampeur (15) qu'après que celuici a atteint sa position extrême avant.

14. Agrafeuse selon la revendication 11, caractérisée en ce que la languette (14) présente une saillie (37) qui actionne un mécanime compteur (33) monté sur le corps (10, 11).

FIG.1

0 085 931

FIG. 2

FIG. 4

FIG. 3

FIG. 5

FIG. 6

FIG. 7

FIG. 8

FIG. 9

FIG. 10

FIG. 11

FIG. 12